Europäisches Patentamt

**European Patent Office**

Office européen des brevets

(19)

(11) Numéro de publication: **0 216 686 B1**

(12) **FASCICULE DE BREVET EUROPEEN**

(45) Date de publication du fascicule du brevet:
21.03.90

(51) Int. Cl.⁴: **A61B 3/08**, A61H 5/00

(21) Numéro de dépôt: 86401955.9

(22) Date de dépôt: 05.09.86

(54) **Dispositif pour le dépistage et le traitement des troubles de la vision binoculaire.**

(30) Priorité: 09.09.85 FR 8513336

(43) Date de publication de la demande:
01.04.87 Bulletin 87/14

(45) Mention de la délivrance du brevet:
21.03.90 Bulletin 90/12

(84) Etats contractants désignés:
BE CH DE GB IT LI NL

(56) Documents cités:
FR-A- 2 489 136
US-A- 3 492 989
US-A- 4 522 474

(73) Titulaire: **Grandière, Brigitte, Route du Buisson St Jean,
F-27000 Evreux(FR)**
Titulaire: **BRIOT INTERNATIONAL, 2, rue Roger Bonnet,
F-27340 Pont de l'Arche(FR)**

(72) Inventeur: **Grandière, Brigitte, Route du Buisson St
Jean, F-27000 Evreux(FR)**
Inventeur: **BRIOT INTERNATIONAL, 2, rue Roger
Bonnet, F-27340 Pont de l'Arche(FR)**

(74) Mandataire: **Fruchard, Guy et al, CABINET
BOETTCHER 23, rue la Boétie, F-75008 Paris(FR)**

## Description

L'invention a pour objet un dispositif destiné à faciliter le dépistage et à aider au traitement des troubles de la vision binoculaire notamment chez les très jeunes enfants. Comme exemple de ces troubles, on peut citer le strabisme et l'amblyopie. Une telle vision se corrige par un traitement médical, orthoptique ou chirurgical. Le traitement orthoptique consiste à rétablir une bonne acuité visuelle monoculaire et, si possible, à développer une vision binoculaire en faisant travailler alternativement, séparément puis simultanément, les yeux du patient.

Un traitement orthoptique est d'autant plus efficace qu'il est mis en œuvre plus précocement, par exemple dès les premiers mois de la vie. Or les moyen matériels d'investigation orthoptiques et ophtalmologiques du nourrisson et du très jeune enfant sont pratiquement inexistants.

On connaît dans l'état de la technique des montures de lunettes munies de verres contenant des cristaux liquides; au moyen d'une polarisation électronique convenable ces verres sont rendus opaques ou transparents, en alternance, de manière indépendante et à un rythme quelconque désiré. Ces lunettes permettent le dépistage, le diagnostic et le traitement de certaines anomalies de la vision, notamment le strabisme.

On connaît également par les documents US-A 3 492 989 et US-A 4 522 474 des dispositifs de traitement de la vision binoculaire dans lesquels les fenêtres d'une monture sont occultées totalement, partiellement indépendamment l'une de l'autre par des éléments mobiles opaques motorisés et dont les mouvements demandent la fourniture d'une énergie et des moyens de commande manuels ou automatiques.

Ces dispositifs perfectionnés, complexes et coûteux à réaliser ne sont pas appropriés au traitement de très jeunes enfants.

L'invention a pour but principal d'apporter un dispositif simple, très économique, ne nécessitant pas de source d'énergie de circuits électroniques perfectionnés, qui est donc utilisable avec les jeunes enfants, même âgés seulement de quelques mois et qui permet un dépistage précoce et sûr des troubles de la vision ainsi qu'un traitement efficace.

A cet effet, l'invention a pour objet un dispositif pour le dépistage et le traitement des troubles de la vision binoculaire tel que défini dans la revendication 1.

La monture a un corps allongé qui présente, de préférence, un volume creux, plus avantageusement encore un volume creux interne inclus dans son épaisseur, et l'élément mobile opaque est monté mobile dans ce volume creux, de préférence à l'aide d'un moyen de support et de guidage en déplacement.

Selon un mode de réalisation de l'invention, l'élément mobile opaque est un volet mobile par pivotement ou par coulissement; en variante il est possible d'équiper le corps de la monture de deux volets capables d'occuper chacun deux positions en relation avec un œil respectif pour obturer ou pour dégager la vision de cet œil.

Selon un autre mode de réalisation, quand le corps de la monture présente un volume creux interne, ce volume est limité par une surface inférieure concave à concavité dirigée vers le haut et l'élément opaque mobile est un corps apte à rouler sur cette surface concave.

Selon un autre mode de réalisation encore, toujours quand la monture présente un volume creux interne, l'élément opaque mobile est un liquide opaque qui remplit partiellement ce volume creux.

Pour bien faire comprendre l'invention, on donnera maintenant, sans intention limitative, une description de plusieurs modes de réalisation de l'invention. On se reportera aux dessins annexés dans lesquels:

- la figure 1 est une vue générale en perspective d'un dispositif de l'invention à volet pivotant,
- la figure 2 est une vue en coupe selon II-II de la figure 1,
- les figures 3 et 4 sont des vues de l'avant montrant des variantes de réalisation du dispositif de l'invention,
- les figures 5 et 6 sont des vues de l'avant montant, à deux positions d'utilisation, un dispositif selon l'invention à élément opaque roulant,
- les figures 7 et 8 sont des vues de l'avant montrant, à deux positions d'utilisation, un dispositif selon l'invention à liquide opaque,
- les figures 9 et 10 sont des vues analogues à la figure 5 montrant chacune, respectivement, une variante à deux volets coulissants utilisant la seule gravité, et une variante à deux volets coulissants utilisant en plus des ressorts de rappel,
- la figure 11 est une vue de l'avant montrant un volet coulissant,
- la figure 12 est une vue de détail relative à deux volets pivotants montés l'un devant l'autre.

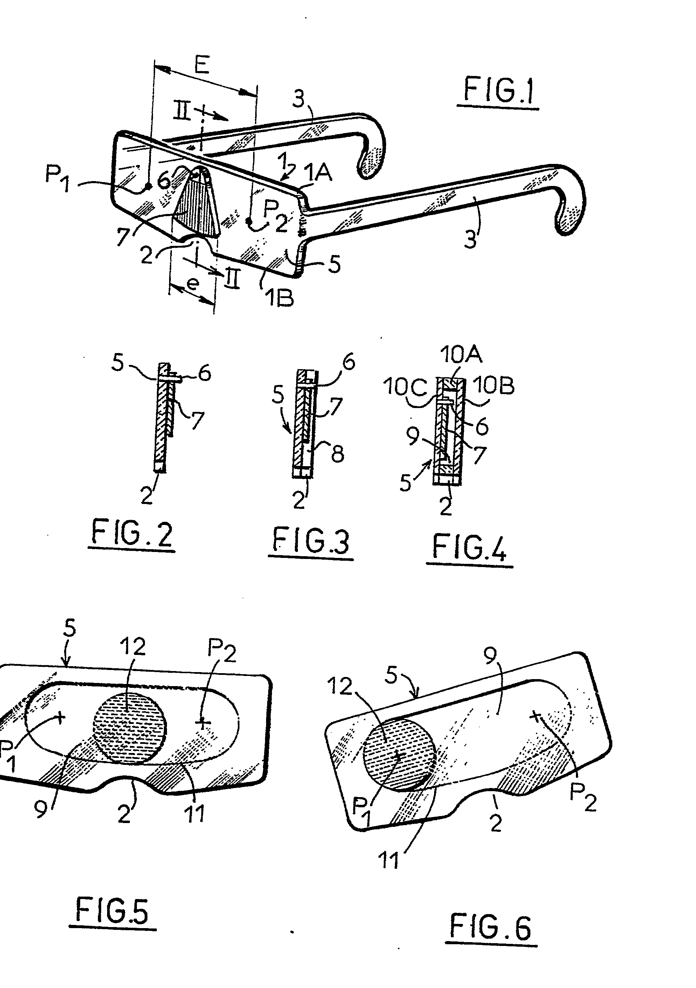

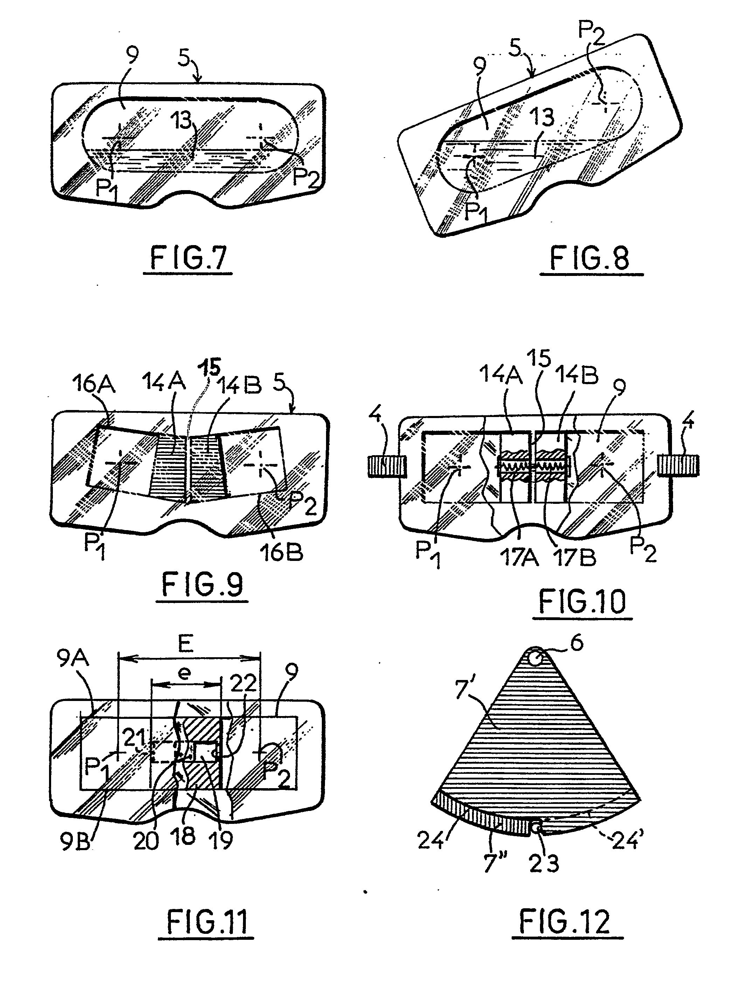

Un dispositif conforme à l'invention comprend une monture 1 réalisée en matière transparente, en totalité ou au moins dans une zone destinée à être placée devant les deux yeux en position d'utilisation. Cette monture 1 se met sur le visage comme la monture d'une paire de lunettes, avec une encoche incurvée 2 dans son bord inférieur 1B pour reposer sur le nez, et avec des moyens de maintien à cette position comme deux branches 3, de préférence pliables (figure 1) ou comme deux cordons 4 (figure 10) se nouant derrière la tête, ou un autre moyen convenable ; ces moyens ont été omis sur les figures 2 à 9 et 11.

Dans l'exemple illustré par les figures 1 et 2, la monture 1 comprend un corps 5 totalement en matière transparente telle que le méthacrylate de méthyle, allongé de façon à s'étendre devant les deux yeux pendant l'utilisation et portant un pivot 6 qui lui est perpendiculaire vers l'avant, c'est-à-dire à l'opposé du visage ; ce pivot 6 est proche du bord supérieur 1A du corps allongé 5 et il est situé dans la zone médiane de ce dernier pour se trouver dans un plan qui est confondu avec le plan de symétrie du visage passant entre les deux yeux quand le dispositif est utilisé. A ce pivot 6 est suspendu un volet 7, analogue à un secteur circulaire centré sur le pivot 6, libre de se déplacer en oscillant d'un côté ou de l'autre quand l'utilisateur du dispositif incline la tête.

Sur les figures, les pupilles d'un utilisateur sont symbolisées par des cercles P1,P2 ; il existe entre ces pupilles P1,P2 un écart pupillaire E (figure 1) à son extrémité la plus large, le volet 7 a une largeur e qui est environ la moitié de l'écart pupillaire E. Quand l'utilisateur incline la tête, le volet 7 est soumis à l'effet de la gravité ; il change de position relative et se trouve devant la pupille P1 ou la pupille P2 en empêchant toute vision par l'oeil correspondant.

La figure 2 montre que le volet pivotant 7 peut être suspendu juste devant le corps 5 ; il est préférable de l'abriter dans un volume creux 8 creusé dans l'épaisseur du corps 5 comme le montre la figure 3 ; il est plus avantageux encore de réaliser le volume creux comme un volume interne 9 inclus dans l'épaisseur du corps 5 ainsi qu'on peut le voir sur la figure 4. Dans ce cas, le corps 5 est composé d'une première plaque centrale 10A à travers laquelle est pratiqué un évidement qui devient le volume interne 9 quand deux plaques pleines avant et arrière 10B et 10C sont fixées, à l'aide d'un adhésif ou d'une soudure, à la plaque centrale 10A. Cette dernière peut être en matière opaque et le volume interne 9 avoir en hauteur et en longueur les dimensions permettant une vision totale à l'aide des deux yeux quand l'utilisateur a la tête droite, le volet 7 occupant alors la position visible sur la figure 1.

Dans les exemples illustrés par les figures 5 à 8, le corps 5 possède un volume interne 9 comme on vient de le décrire. Dans le cas des figures 5 et 6, ce volume interne 9 est limité par une surface inférieure 11 qui est concave avec sa concavité tournée vers le haut en position d'utilisation et l'élément mobile opaque est un élément 12 apte à rouler sur la surface concave 11, par exemple un disque en matière opaque contenu librement dans le volume interne 9. En position de vision normale binoculaire, l'élément 12 occupe le point le plus bas de la surface concave 11, point qui est dans le plan de symétrie du visage. Quand la tête est inclinée (figure 6), l'élément opaque 12 vient se placer devant une pupille P1; la largeur du volume interne 9 est déterminée à cet effet ainsi que la grosseur de l'élément roulant 12.

Dans le cas des figures 7,8, le volume interne 9 contient un liquide opaque 13 ; en position de vision binoculaire ce liquide 13 s'étale dans la zone inférieure du volume 9 et ne gêne pas la vision (figure 7). Quand la tête est inclinée, le liquide 13 se rassemble dans une partie extrême du volume interne 9 et bouche la vue d'un œil.

Il est possible d'utiliser deux liquides non miscibles, l'un plus dense, l'autre plus léger, pour remplir complètement le volume interne 9. De même, dans l'exemple des figures 5,6 le volume 9 peut être rempli totalement d'un liquide transparent dans lequel peut baigner l'élément roulant 12. Ainsi les mouvements soit du liquide opaque dense, soit de l'élément roulant sont freinés légèrement ; ils sont plus lents et plus réguliers.

Dans l'exemple illustré par la figure 9, le dispositif comprend deux éléments mobiles 14A,14B, opaques, indépendants, qui sont disposés de part et d'autre d'une butée centrale 15 située dans le plan de symétrie du visage, pendant l'utilisation. Chaque élément mobile 14A,14B est supporté et guidé par un moyen convenable qui peut être une paire de glissières 16A,16B respectivement, qui s'étendent à partir de la butée centrale 15, d'un côté et de l'autre de celle-ci. Ces glissières 16A,16B pourraient être en relief sur une face du corps 5 ; en variante elle peuvent être constituées par les faces supérieure 9A et inférieure 9B du volume interne 9. Ces volets 16A,16B sont des volets coulissant dans le sens longitudinal de la monture 1 entre deux positions : une position où ils sont appuyés tous les deux contre la butée centrale 15 et permettent la vision binoculaire et une position où l'un des volets a glissé jusqu'à venir se placer à l'extrémité de ses glissières, devant une pupille. Pour que chaque volet 14A,14B tende à venir s'appuyer contre la butée centrale 15 sous l'effet de la gravité, il faut donner aux glissières 16A,16B une faible inclinaison vers la butée centrale 15, comme le montre la figure 9.

La figure 10 se rapporte à une variante de réalisation dans laquelle les volets coulissants 14A,14B sont supportés et guidés en translation dans un volume interne 9 dont les surfaces supérieure et inférieure constituent les glissières 16A,16B ; ces dernières sont en prolongement direct les unes des autres et horizontales en position de vision binoculaire. Chaque volet coulissant 14A,14B s'éloigne en coulissant de la butée centrale 15 sous l'effet de la gravité, quand le dispositif est incliné, contre l'action d'un ressort de rappel 17A,17B qui le relie à la butée centrale 15.

La figure 11 montre une autre variante comprenant un unique volet coulissant 18, rectangulaire, ayant sur une face principale une rainure rectiligne 19 dans laquelle est contenue une tête de guidage 20. Cette dernière est en relief au centre du volume interne 9 du corps 5. La course du volet coulissant 18 est limitée par des butées extrêmes 21,22 prévues aux extrémités de la rainure 19. Ce volet 18 a une longueur e qui est égale à la moitié de l'écart pupillaire E.

Dans l'exemple de la figure 11, le volet 18 est guidé intérieurement par la tête de guidage et de support 20. Il pourrait être guidé et supporté extérieurement par les faces supérieure 9A et inférieure 9B du volume interne 9, la tête de guidage 20 étant supprimée.

En revenant à la figure 2, on constatera que le corps 5 pourrait être en matière transparente avec la tête de guidage 20 en relief sur une des faces principales et le volet coulissant 18 porté et guidé par cette tête, celle-ci et la rainure 20 ayant alors des profils en T complémentaires.

Les exemples des figures 1, 5 et 11 pourraient comporter deux éléments mobiles opaques comme le volet pivotant 7 ou l'élément roulant 12 ou le volet coulissant 18 disposés l'un devant l'autre en position de vision binoculaire. Chacun d'eux se déplacerait entre deux positions seulement comme les volets des figures 9 et 10 par rapport à une butée centrale commune 23 comme le montre la figure 12 pour deux volets pivotants 7',7", ces derniers ayant un bord découpé partiellement en 24,24' respectivement pour permettre un déplacement dans un sens seulement.

Le dispositif de l'invention permet de dépister certains troubles de la vision des enfants et des petits enfants par simple observation de leur comportement, sans manoeuvre ou manipulation exécutée par une tierce personne.

Quand un enfant souffre d'un trouble de la vision binoculaire, il a tendance à ne pas garder la tête en position droite mais à la pencher d'un côté afin d'avoir une vision monoculaire. L'élément opaque mobile se déplace en conséquence et indique par sa position l'oeil déficient.

Pour le traitement des troubles, suivant les mouvements de la tête de l'enfant, l'élément opaque mobile se déplace à gauche et à droite et permet l'alternance de la vision. Le dispositif évite ainsi le développement d'une amblyopie.

En outre, en raison de l'alternance de la vision due au comportement de l'enfant, le dispositif sollicite aussi la fixation centrale de la vue et favorise le developpement de la vision binoculaire en position droite de la tête.

**Revendications**

1. Dispositif pour le dépistage et le traitement des troubles de la vision binoculaire d'un patient, comprenant une monture (1) transparente au moins dans une zone destinée à être placés devant les deux yeux, en position d'utilisation, et des moyen (3, 4) de fixation de cette monture à cette position sur la tête du patient, cette monture (1) comprenant des moyens de support et de guidage en déplacement (6, 11, 16A, 16B, 20) fixés à ladite monture (1) pour au moins un élément opaque (7, 12, 13, 14A, 14B, 18) mobile entre deux positions au moins: une position où cet élément opaque laisse libre la vision binoculaire, et une position où il empêche la vision à l'aide de l'un des deux yeux, caractérisé en ce que ledit élément opaque (7, 12, 13, 14A, 14B, 18) est mobile sous l'effet de la gravité lors des mouvements de la tête du patient.

2. Dispositif selon la revendication 1, caractérisé en ce qu'il comprend un unique élément mobile opaque (7, 12, 13, 18) déplaçable entre trois positions: une position où il laisse libre la vision binoculaire, une position où il empêche la vision à l'aide d'un œil, et une position où il empêche la vision à l'aide de l'autre œil.

3. Dispositif selon la revendication 1, caractérisé en ce qu'il comprend deux éléments mobiles opaques (14A, 14B, 7', 7") déplaçables chacun entre deux positions; une position où chaque élément laisse libre la vision binoculaire, et une position où chaque élément empêche respectivement la vision à l'aide d'un œil.

4. Dispositif selon la revendication 3, caractérisé en ce que les deux éléments mobiles opaques (14A, 14B) sont disposés symétriquement de part et d'autre d'une butée centrale (15) portée par la monture (1), cette butée centrale (15) étant, en position d'utilisation du dispositif, dans le plan de symétrie du visage du patient.

5. Dispositif selon la revendication 3, caractérisé en ce que les deux éléments mobiles opaques (7', 7") sont disposés l'un devant l'autre à leur position laissant libre la vision binoculaire, au moins une butée (23) limitant le mouvement de chaque élément respectivement dans des sens opposés.

6. Dispositif selon la revendication 1, caractérisé en ce que la monture a un corps allongé (5) qui présente un volume creux (9) dans lequel est monté à l'aide du moyen de support et de guidage en déplacement (6, 11, 20) ledit élément mobile (7, 12, 13).

7. Dispositif selon la revendication 6, caractérisé en ce que le volume creux est un volume interne (9) inclus dans l'épaisseur de la monture (1).

8. Dispositif selon la revendication 7, caractérisé en ce que l'élément opaque mobile est un élément opaque (12) apte à rouler inclus dans le volume interne (9), ce dernier étant limité par une surface inférieure (11) ayant en position d'utilisation un profil concave tourné vers le haut avec son point le plus bas situé sensiblement à égale distance des deux yeux, l'élément opaque (12) pouvant rouler librement sur cette surface (11).

9. Dispositif selon l'une quelconque des revendications 1 ou 2, caractérisé en ce que l'élément mobile opaque est un volet (7) monté pivotant autor d'un pivot (6) supporté perpendiculairement par la monture (1) en un point situé sensiblement dans le plan de symétrie du visage pendant l'utilisation.

10. Dispositif selon la revendication 7, caractérisé en ce que l'élément mobile opaque est un liquide (13) enfermé dans le volume interne (9).

11. Dispositif selon l'une quelconque des revendications 1, 2, 6, 7 caractérisé en ce que l'élément mobile opaque est un volet (18) monté coulissant dans le sens longitudinal de la monture (1).

**Claims**

1. Apparatus for detecting and treating troubles with binocular vision of a patient, the apparatus comprising a frame (1) transparent at least in a region thereof intended to be placed directly in front of the eyes and means (3, 4) for holding said frame on the patient's head in such an inuse position, said frame (1) including support and guide means (6, 11, 16A, 16B, 20) fixed to the frame (1) for supporting at least one moving opaque element (7, 12, 13, 14A, 14B, 18) and for guiding movement thereof relative to said frame between at least two positions: a first position in which said opaque element allows free binocular vision; and a second position in which it prevents vision with one or other of the eyes, characterized in that said opaque element (7, 12, 13, 14A, 14B, 18) is movable under the effect of the gravity when the patient moves his head.

2. Apparatus according to claim 1, characterized in that a single moving opaque element (7, 12, 13, 18) is movable between three positions: a first position in which it allows free binocular vision; a second position in which it prevents vision using one eye; and a third position in which it prevents vision using the other eye.

3. Apparatus according to claim 1, characterized in that two opaque moving (14A, 14B, 7', 7") elements are provided, each displaceable between two positions: i.e. respective first positions in which binocular vision is left free; and respective second positions in which vision using a corresponding one of the eyes is prevented.

4. Apparatus according to claim 3, characterized in that said two opaque elements (14A, 14B) are disposed symmetrically on either side of a center stop (15) on said frame (1), said center stop (15) being located in the plane of symmetry of the patient's face when the apparatus is in use.

5. Apparatus according to claim 3, characterized in that said two moving opaque elements (7', 7'') are disposed one in front of the other in their respective first positions allowing free binocular vision, with at least one stop (23) limiting movement of each element in respective opposite directions.

6. Apparatus according to claim 1, characterized in that the frame includes an elongated body (5) having a hollow volume (9) in which said moving element (7, 12, 13) is mounted by said support and guide means (6, 11, 20).

7. Apparatus according to claim 6, characterized in that said hollow volume is an internal volume (9) included in the thickness of the frame (1).

8. Apparatus according to claim 7, characterized in that the moving opaque element (12) is suitable for rolling and is included within said internal volume (9), said internal volume being delimited in part, by a bottom surface (11) having a concave face which is upwardly directed when the apparatus is in use, with the lowest point of said concave surface being substantially equidistant between the eyes, said opaque element (12) being capable of rolling freely on this surface (11).

9. Apparatus according to claim 1 or 2, characterized in that the moving opaque element is a shutter (7) mounted to pivot about a pivot (6) extending perpendicularly to the frame (1) at a point situated substantially in the plane of symmetry of the face when the apparatus is in use.

10. Apparatus according to claim 7, characterized in that the moving opaque element is a liquid (13) enclosed in the internal volume (9).

11. Apparatus according to any one of claims 1, 2, 6, or 7, characterized in that the moving opaque element is a shutter (18) mounted to slide longitudinally relative to the frame (1).

**Patentansprüche**

1. Vorrichtung zur Feststellung und Behandlung von Störungen des Binokularsehens eines Patienten, umfassend eine Fassung (1), die wenigstens in einer in einer Gebrauchsstellung vor den beiden Augen anzuordnenden Zone transparent ist, und Mittel (3, 4) zum Fixieren dieser Fassung in dieser Stellung auf dem Kopf eines Patienten, wobei die Fassung (1) Halte- und Führungsmittel für eine Verlagerung (6, 11, 16A, 16B, 20) umfaßt, welche an der Fassung (1) für weinigstens ein lichtundurchlässiges Element (7, 12, 13, 14A, 14B, 18) befestigt sind, das wenigstens zwischen zwei Positionen bewegbar ist: eine Position, in der das lichtundurchlässige Element das Binokularsehen freigibt, und eine Position, in der es das Sehen mit Hilfe eines der beiden Augen verhindert, dadurch gekennzeichnet, daß das lichtundurchlässige Element (7, 12, 13, 14A, 14B, 18) unter dem Einfluß der Schwerkraft infolge Bewegungen des Kopfes des Patienten beweglich ist.

2. Vorrichtung nach Anspruch 1, dadurch gekennzeichnet, daß sie ein einziges bewegliches, lichtundurchlässiges Element (7, 12, 13, 18) umfaßt, das zwischen drei Positionen verschiebbar ist: eine Position, in der es das Binokularsehen freigibt, eine Position, in der es das Binokularsehen mit Hilfe des einen Auges verhindert, und eine Position, in der es das Sehen mit Hilfe des anderen Auges verhindert.

3. Vorrichtung nach Anspruch 1, dadurch gekennzeichnet, daß sie zwei bewegliche, lichtundurchlässige Elemente (14A, 14B, 7', 7'') umfaßt, von denen jedes zwischen zwei Positionen verschiebbar ist: eine Position, in der jedes Element das Binokularsehen freigibt, und eine Stellung, in der jedes Element das Sehen mit Hilfe des jeweiligen einen Auges verhindert.

4. Vorrichtung nach Anspruch 3, dadurch gekennzeichnet, daß die zwei beweglichen, lichtundurchlässigen Elemente (14A, 14B) symmetrisch beidseits eines von der Fassung (1) getragenen zentralen Anschlags (15) angeordnet sind, wobei sich der zentrale Anschlag (15) in der Gebrauchsstellung der Vorrichtung in der Symmetrieebene des Gesichtes des Patienten befindet.

5. Vorrichtung nach Anspruch 3, dadurch gekennzeichnet, daß die zwei beweglichen, lichtundurchlässigen Elemente (7', 7'') in ihrer das Binokularsehen freigebenden Stellung hintereinander angeordnet sind, wobei mindestens ein Anschlag (23) die Bewegung jedes Elements jeweils in entgegengesetzte Richtungen begrenzt.

6. Vorrichtung nach Anspruch 1, dadurch gekennzeichnet, daß die Fassung einen länglichen Körper (5) hat, der ein Hohlvolumen (9) aufweist, in welchem das bewegliche Element (7, 12, 13) mit Hilfe des Halte- und Führungsmittels zum Verschieben (6, 11, 20) befestigt ist.

7. Vorrichtung nach Anspruch 6, dadurch gekennzeichnet, daß das Hohlvolumen ein in der Dicke der Fassung (1) eingeschlossenes Innenvolumen (9) ist.

8. Vorrichtung nach Anspruch 7, dadurch gekennzeichnet, daß das lichtundurchlässige, bewegliche Element ein lichtundurchlässiges Element (12) ist, das eingeschlossen in dem Innenvolumen (9) rollen kann, wobei letzteres durch eine innere Fläche (11) begrenzt ist, welche in der Gebrauchsstellung ein nach oben gekrümmtes konkaves Profil hat, dessen unterster Punkt von beiden Augen mit im wesentlichen derselben Entfernung angeordnet ist, wobei das lichtundurchlässige Element (12) auf dieser Fläche (11) frei rollen kann.

9. Vorrichtung nach einem der Ansprüche 1 oder 2, dadurch gekennzeichnet, daß das bewegliche, lichtundurchlässige Element eine Verschlußklappe (7) ist, die um eine Schwenkachse schwenkbar gelagert ist, welche senkrecht zur Fassung (1) in einem Punkt gelagert ist, der während des Gebrauchs im wesentlichen in der Symmetrieebene des Gesichts liegt.

10. Vorrichtung nach Anspruch 7, dadurch gekennzeichnet, daß das bewegliche, lichtundurchlässige Element eine in dem Innenvolumen (9) eingeschlossene Flüssigkeit (13) ist.

11. Vorrichtung nach einem der Ansprüche 1, 2, 6 oder 7, dadurch gekennzeichnet, daß das bewegliche, lichtundurchlässige Element eine Verschlußklappe (18) ist, die in Längsrichtung der Fassung (1) verschiebbar gelagert ist.

FIG.1

FIG.2

FIG.3

FIG.4

FIG.5

FIG.6

FIG.7

FIG.8

FIG.9

FIG.10

FIG.11

FIG.12